# EUROPEAN PATENT APPLICATION

(11) **EP 4 303 881 A1**
(43) Date of publication of application: **10.01.2024**
(21) Application number: 23170651.6
(22) Date of filing: 28.04.2023
(51) Int. Cl.: G16H 20/00, G16H 40/20, G16H 50/70

(54) **MEDICAL INFORMATION PROVIDING METHOD, MEDICAL INFORMATION PROVIDING PROGRAM, AND INFORMATION PROCESSING APPARATUS**

(30) Priority: 08.07.2022 JP 2022110407
(71) Applicant: FUJITSU LIMITED, Kawasaki-shi, Kanagawa 211-8588 (JP)
(72) Inventor: MIZOUCHI, Tsuyoshi, Kawasaki-shi, Kanagawa, 211-8588 (JP); AMEMIYA, Satoshi, Kawasaki-shi, Kanagawa, 211-8588 (JP); KURAKI, Kensuke, Kawasaki-shi, Kanagawa, 211-8588 (JP); INOMATA, Akihiro, Kawasaki-shi, Kanagawa, 211-8588 (JP); HOTTA, Shinji, Kawasaki-shi, Kanagawa, 211-8588 (JP)
(74) Representative: Hoffmann Eitle

(57) **Abstract**

A medical information providing method including: acquiring first injury-or-disease information that indicates a type of an injury-or-disease; accessing a storage device that stores, for each provider of a plurality of providers each of which provides medical service, identification information of the provider, and injury-or-disease treatment information, the injury-or-disease treatment information including information on a type of an injury-or-disease for which the provider has an experience of treatment or that the provider is able to treat, and information on a type of treatment; extracting, for each provider of the plurality of providers, from the storage device, the injury-or-disease treatment information that includes information on a type of injury-or-disease corresponding to the first injury-or-disease information; and in a case where the information on a type of treatment included in the injury-or-disease treatment information extracted for each provider of the plurality of providers satisfies a criterion, outputting the identification information of the provider.

## Description

### FIELD

The embodiments discussed herein are related to a medical information providing method, a medical information providing program, and an information processing apparatus.

### BACKGROUND

In local governments such as prefectures, cities, towns, and villages, a medical policy in which doctors, medical institutions, and medical care departments (referred to as doctors and the like) provide services in cooperation with each other has been planned and implemented in order to enable residents to receive high-quality medical care at ease.

In a case where doctors and the like provide services in cooperation with each other, a doctor or the like may introduce another doctor or the like to the patient, and at the time of introduction, it is desirable to select a doctor or the like suitable for the patient. A technique for enabling a medical institution to efficiently search for another medical institution at the time of such introduction is known. A technique is disclosed in which the degree of cooperation between medical institutions is indexed based on the number of times that a medical institution introduces another medical institution obtained from a receipt database.

Japanese Laid-open Patent Publication No. 2013-89056 is disclosed as related art.

### SUMMARY

### TECHNICAL PROBLEMS

In a case where a doctor introduces another doctor to a patient, it is important that the other doctor is capable of treating an injury or disease of the patient. However, in the medical institution search system of Japanese Laid-open Patent Publication No. 2013-89056, the kinds of treatments that may be performed by each doctor or medical institution are not taken into consideration.

In one aspect, an object of the present disclosure is to provide a medical information providing method and a medical information providing program capable of outputting information on a medical service provider capable of treating an injury or disease, and an information processing apparatus.

### SOLUTION TO PROBLEM

According to an aspect of the embodiments, there is provided a medical information providing method implemented by a computer, the medical information providing method including: acquiring first injury-or-disease information that indicates a type of an injury-or-disease; accessing a storage device that stores, for each provider of a plurality of providers each of which provides medical service, identification information of the provider, and injury-or-disease treatment information, the injury-or-disease treatment information including information on a type of an injury-or-disease for which the provider has an experience of treatment or that the provider is able to treat, and information on a type of treatment; extracting, for each provider of the plurality of providers, from the storage device, the injury-or-disease treatment information that includes information on a type of injury-or-disease corresponding to the first injury-or-disease information; and in a case where the information on a type of treatment included in the injury-or-disease treatment information extracted for each provider of the plurality of providers satisfies a given criterion, outputting the identification information of the provider.

### ADVANTAGEOUS EFFECTS OF INVENTION

Information on a medical service provider capable of treating an injury or disease may be output.

### BRIEF DESCRIPTION OF DRAWINGS

FIG. 1 is a diagram schematically illustrating a configuration of a medical information providing system according to a first embodiment;
FIG. 2A is a diagram illustrating an example of a hardware configuration of a doctor terminal, and FIG. 2B is a diagram illustrating an example of a hardware configuration of a server;
FIG. 3 is a functional block diagram of the server;
FIG. 4A is a diagram illustrating an example of medical examination history data of the first embodiment, and FIG. 4B is a diagram illustrating an example of a correspondence table of the first embodiment;
FIG. 5 is a flowchart illustrating an example of processing of the server;
FIG. 6 is a diagram illustrating an example of a screen displayed on a display unit of the doctor terminal;
FIG. 7 is a diagram illustrating a state in which the identification information of specialists a and b as introduction candidates is displayed on a screen;
FIG. 8 is a diagram illustrating another example of screen display;
FIG. 9 is a flowchart illustrating processing of the server of Modification Examples 1 and 3;
FIGs. 10A and 10B are diagrams illustrating correspondence tables used in Modification Examples 2 and 3;
FIGs. 11A and 11B are diagrams for explaining Modification Example 4 (part 1);
FIG. 12 is a diagram for explaining Modification Example 4 (part 2);
FIG. 13 is a diagram for explaining Modification Example 5 (part 1);
FIG. 14 is a diagram for explaining Modification Example 5 (part 2);
FIGs. 15A and 15B are diagrams illustrating examples of screen display of Modification Example 6;
FIG. 16A is a diagram illustrating an example of medical examination history data of a second embodiment, and FIG. 16B is a diagram illustrating an example of a correspondence table of the second embodiment;
FIGs. 17A and 17B are diagrams illustrating examples of screens displayed on the display unit of the doctor terminal in the second embodiment;
FIG. 18A is a diagram illustrating an example of medical examination history data of a third embodiment, and FIG. 18B is a diagram illustrating an example of a correspondence table of the third embodiment; and
FIG. 19 is a diagram illustrating an example of a screen displayed on the display unit of the doctor terminal in the third embodiment.

### DESCRIPTION OF EMBODIMENTS

### <<First Embodiment>>

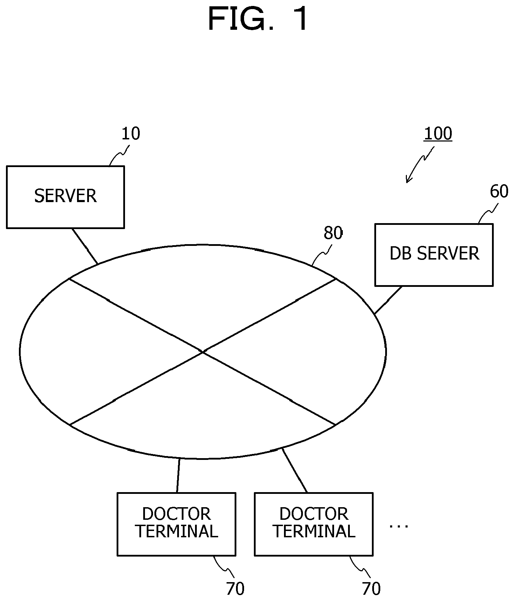

Hereinafter, a first embodiment will be described in detail based on FIGs. 1 to 8.

FIG. 1 schematically illustrates a configuration of a medical information providing system 100 according to the first embodiment. The medical information providing system 100 of the present embodiment is a system that provides an attending doctor of a patient with information that makes it easy to select a specialist to be introduced, for example, in a situation where the attending doctor is unable to treat an injury or disease of the patient and introduces a specialist of a cooperation destination to the patient.

As illustrated in FIG. 1, the medical information providing system 100 includes a plurality of doctor terminals 70, a server 10 serving as an information processing apparatus, and a database (DB) server 60 serving as a storage unit. The doctor terminal 70, the server 10, and the DB server 60 are coupled to a network 80 such as the Internet or a local area network (LAN), and data may be exchanged between the apparatuses.

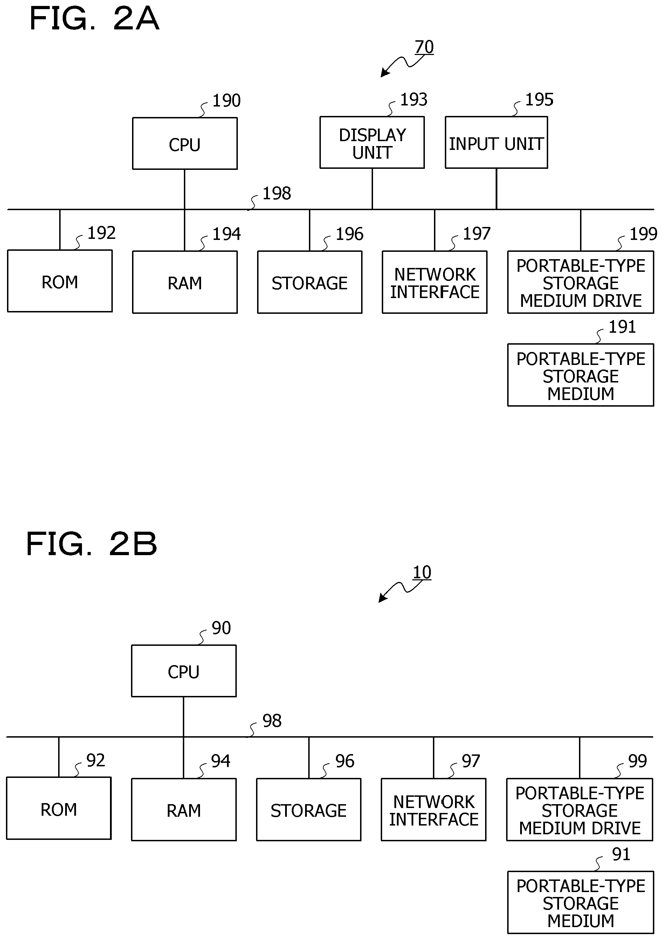

The doctor terminal 70 is a terminal such as a personal computer (PC) or a smartphone usable by each doctor. FIG. 2A illustrates a hardware configuration of the doctor terminal 70. As illustrated in FIG. 2A, the doctor terminal 70 includes a central processing unit (CPU) 190, a read-only memory (ROM) 192, a random-access memory (RAM) 194, a storage (in this case, a solid-state drive (SSD), a hard disk drive (HDD), or the like) 196, a network interface 197, a display unit 193, an input unit 195, a portable-type storage medium drive 199, and the like. These components of the doctor terminal 70 are coupled to a bus 198. The display unit 193 includes a liquid crystal display or the like, and the input unit 195 includes a keyboard, a mouse, a touch panel, and the like. In the doctor terminal 70, the CPU 190 executes a program stored in the ROM 192 or the storage 196 or a program read from a portable-type storage medium 191 by the portable-type storage medium drive 199.

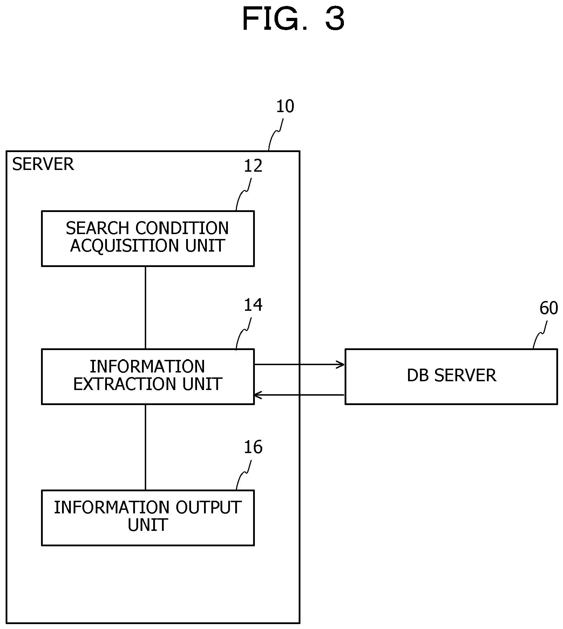

Returning to FIG. 1, the server 10 is an information processing apparatus that searches for and extracts information stored in the DB server 60 using search conditions of a doctor received from the doctor terminal 70, and transmits information based on the extracted information to the doctor terminal 70. FIG. 2B illustrates a hardware configuration of the server 10. As illustrated in FIG. 2B, the server 10 includes a CPU 90, a ROM 92, a RAM 94, a storage 96, a network interface 97, a portable-type storage medium drive 99, and the like. These components of the server 10 are coupled to a bus 98. In the server 10, the CPU 90 executes a program (including a medical information providing program) stored in the ROM 92 or the storage 96 or a program read from a portable-type storage medium 91 by the portable-type storage medium drive 99, thereby realizing the function as each unit illustrated in FIG. 3. For example, the function of each unit in FIG. 3 may be realized by an integrated circuit, such as an application-specific integrated circuit (ASIC) or a field-programmable gate array (FPGA).

FIG. 3 illustrates a functional block diagram of the server 10. In the server 10, the CPU 90 executes a program, thereby realizing the functions of a search condition acquisition unit 12 as an acquisition unit, an information extraction unit 14 as an extraction unit, and an information output unit 16 as an output unit.

The search condition acquisition unit 12 acquires the search conditions input in the doctor terminal 70 and notifies the information extraction unit 14 of the search conditions. The search conditions include information on a doctor who has input the search conditions, information on a doctor of a cooperation destination, and an injury or disease name of a patient.

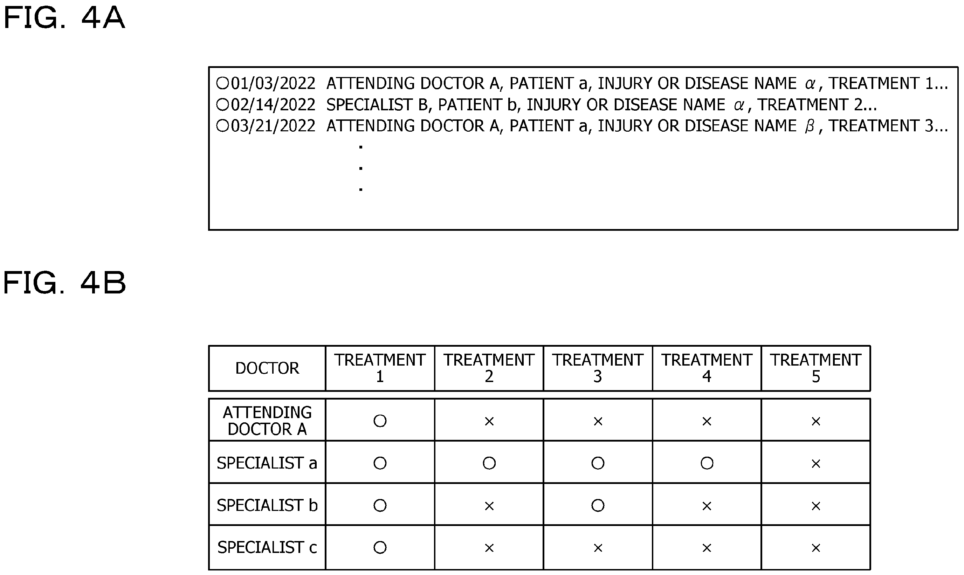

The information extraction unit 14 searches for information stored in the DB server 60 based on the search conditions. Information generated each time a doctor treats a patient, for example, medical examination history data of a patient created in past medical examinations is stored in the DB server 60. FIG. 4A illustrates an example of medical examination history data (partial excerpt). Each record (row) of medical examination history data includes information such as year, month, and day of medical examination (treatment), identification information of a doctor who has performed the medical examination (treatment), identification information of a patient, an injury or disease name, and a type of the performed treatment. For example, in medical examination history data, in association with the identification information of a doctor, injury or disease treatment information including information on the type of injury or disease for which the doctor has an experience of treatment and information on the type of treatment, and information on the date on which the treatment was performed are stored. When information on a doctor and an injury or disease name are input as search conditions, the information extraction unit 14 searches for and extracts data (record) created when the doctor of a search target performed the treatment for the injury or disease of a search target, from the medical examination history data. The information extraction unit 14 creates a correspondence table (see FIG. 4B) based on the contents of the extracted record. In each field of the correspondence table in FIG. 4B, "∘" is stored if each doctor has an experience of performing each treatment, and "×" is stored if each doctor has no experience of performing each treatment.

Based on the correspondence table created by the information extraction unit 14, the information output unit 16 determines whether the type of treatment that has been performed by each doctor of a cooperation destination (type of experienced treatment) satisfies a criterion. The information output unit 16 generates a screen for displaying information on a doctor of a cooperation destination satisfying the criterion, and transmits (outputs) the screen to the doctor terminal 70.

### (Processing of Server 10)

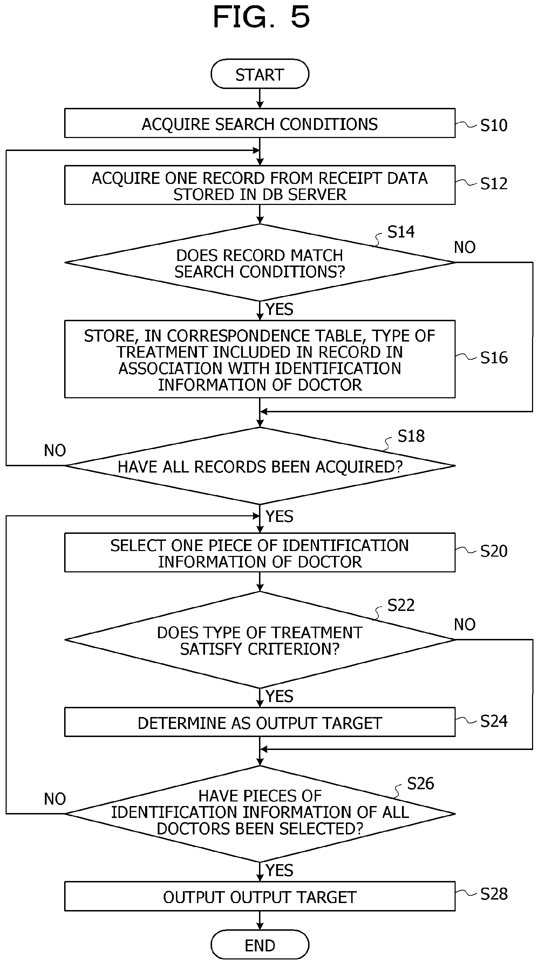

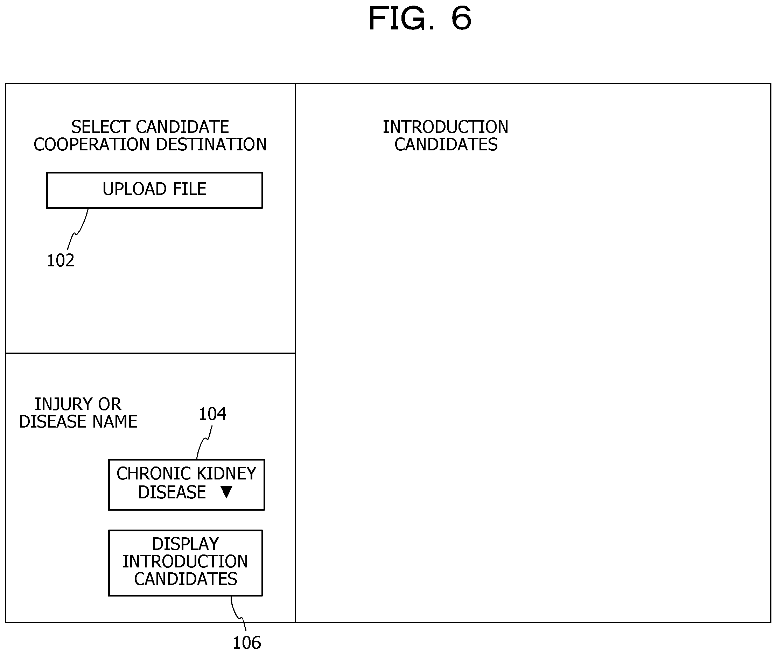

FIG. 5 is a flowchart illustrating processing of the server 10. As a premise for the processing in FIG. 5 to be started, a screen such as that illustrated in FIG. 6 is displayed on the display unit 193 of the doctor terminal 70. The screen in FIG. 6 may be displayed on a browser or may be displayed by starting a dedicated application. The doctor who has caused the screen in FIG. 6 to be displayed is attending doctor A of a patient. Attending doctor A causes the screen in FIG. 6 to be displayed in order to search for a specialist to request treatment for an injury or disease of the patient (for example, a specialist to be introduced to the patient).

When the processing in FIG. 5 is started, first, in step S10, the search condition acquisition unit 12 acquires search conditions. Attending doctor A uploads a file in which the identification information of doctors of cooperation destinations is stored by pressing an "upload file" button 102 on the screen in FIG. 6. Attending doctor A selects an injury or disease name of the patient from a drop-down list (pull-down) 104, and presses a "display introduction candidates" button 106. In this case, the doctor terminal 70 transmits, to the search condition acquisition unit 12 of the server 10, a list of the identification information of doctors of cooperation destinations included in the uploaded file and the information on the selected injury or disease name (first injury or disease information). The doctor terminal 70 also transmits the identification information of attending doctor A to the search condition acquisition unit 12 of the server 10. The search condition acquisition unit 12 acquires, as search conditions, the list of the identification information of doctors of cooperation destinations and the identification information of attending doctor A, and the information on the selected injury or disease name, which are transmitted from the doctor terminal 70.

Next, when the processing proceeds to step S12, the information extraction unit 14 acquires one record from the medical examination history data (FIG. 4A) stored in the DB server 60.

Next, in step S14, the information extraction unit 14 determines whether the acquired record matches the search conditions. For example, the information extraction unit 14 determines whether the identification information of a doctor in the acquired record is the identification information of a doctor of a cooperation destination or the identification information of attending doctor A, and the injury or disease name in the acquired record matches the injury or disease name selected by attending doctor A. When the determination in step S14 is affirmative, the information extraction unit 14 proceeds to step S16.

When the processing proceeds to step S16, the information extraction unit 14 stores, in a correspondence table (FIG. 4B), the type of treatment included in the record in association with the identification information of a doctor. For example, when the first record in FIG. 4A matches the search conditions, "o" is stored in the field of treatment 1 of attending doctor A. After the processing in step S16 is performed, the processing proceeds to step S18. When the determination in step S14 is negative, the processing proceeds to step S18 without going through step S16.

When the processing proceeds to step S18, the information extraction unit 14 determines whether all records have been acquired. When the determination in step S18 is negative, the processing returns to step S12 and a record that has not been acquired is acquired, and the processing of step S14 and subsequent steps described above is repeated. On the other hand, when the determination in step S18 is affirmative, the processing proceeds to step S20. At the stage of proceeding to step S20, the correspondence table in FIG. 4B is completed.

When the processing proceeds to step S20, the information output unit 16 selects one piece of identification information of a doctor other than attending doctor A in the correspondence table. For example, specialist a in FIG. 4B is selected.

Next, in step S22, the information output unit 16 determines whether the type of treatment of the selected doctor satisfies a criterion. As an example, the "criterion" in this case is that "the treatments of the selected doctor include a treatment other than the treatments of the doctor (attending doctor A) who has input the search conditions". The "criterion" is not limited to the above example, and may be that "the selected doctor is capable of performing at least one treatment", "the treatments of the selected doctor include at least two treatments other than the treatments of the doctor (attending doctor A) who has input the search conditions", or the like. When the determination in step S22 is affirmative, the information output unit 16 proceeds to step S24 and determines the identification information of the selected doctor as an output target. After step S24 is performed, the information output unit 16 proceeds to step S26. On the other hand, when the determination in step S22 is negative, the information output unit 16 proceeds to step S26 without going through step S24.

When the processing proceeds to step S26, the information output unit 16 determines whether the pieces of identification information of all doctors have been selected. When the determination in step S26 is negative, the processing returns to step S20, and the processing and determination in steps S20 to S26 are repeatedly executed. When the determination in step S26 is affirmative, the information output unit 16 proceeds to step S28.

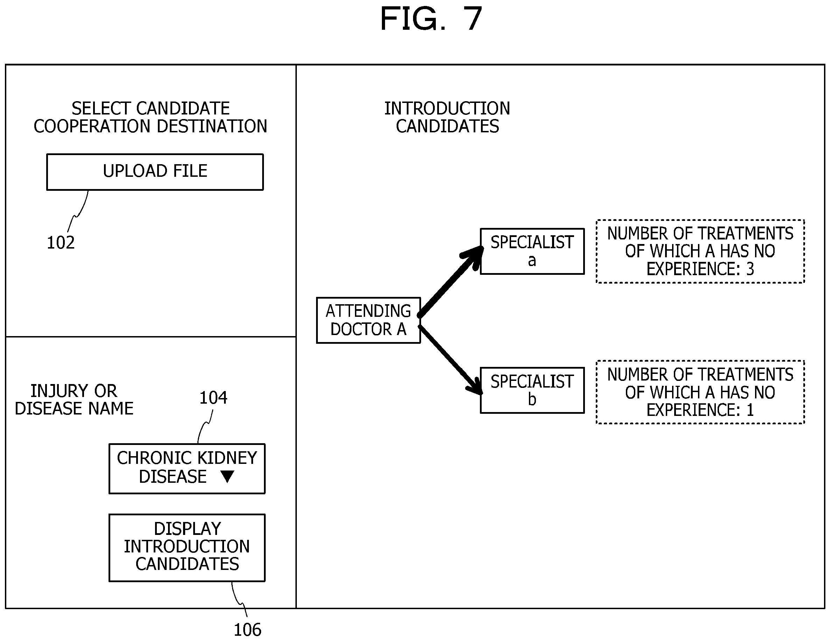

When the processing proceeds to step S28, the information output unit 16 outputs the output target to the doctor terminal 70. For example, in a case where the "criterion" is that "the treatments of the selected doctor include a treatment other than the treatments of the doctor (attending doctor A) who has input the search conditions", specialists a and b are output targets according to FIG. 4B. In this case, as illustrated in FIG. 7, the information output unit 16 displays the identification information of specialists a and b as introduction candidates on the screen. The information output unit 16 may simply display only the identification information of specialists a and b, but as illustrated in FIG. 7, the information output unit 16 may display the number of types of treatments of which attending doctor A has no experience, or may perform display according to the number of types of treatments of which attending doctor A has no experience. As the display according to the number of types of treatments of which attending doctor A has no experience, for example, as illustrated in FIG. 7, the degree of priority (degree to which priority is to be given as an introduction candidate) may be represented by the thickness of an arrow. The degree of priority may be represented by rearranging the displayed order of identification information of doctors in descending order of the number of types of treatments of which attending doctor A has no experience.

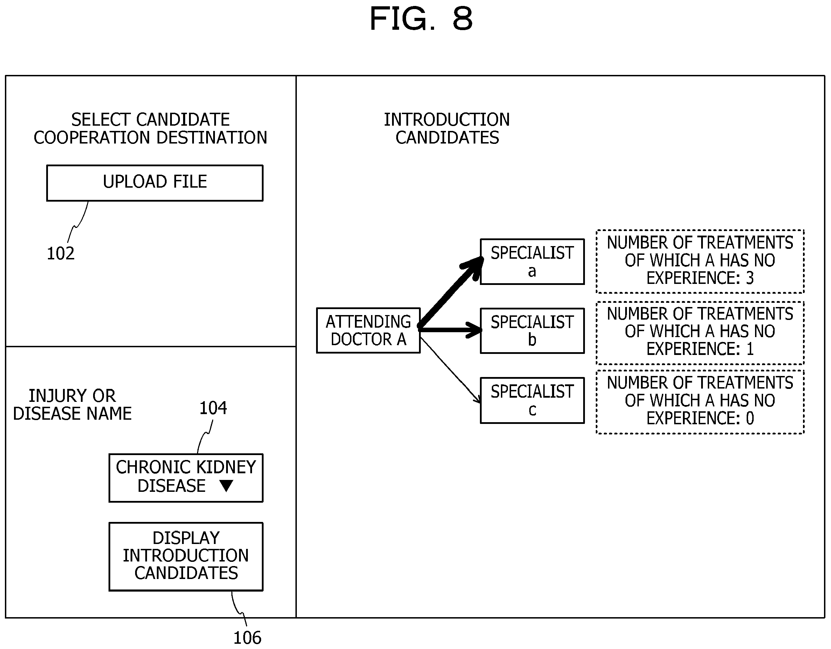

For example, in a case where the "criterion" is that "the selected doctor is capable of performing at least one treatment", specialists a, b, and c are output targets according to FIG. 4B. In this case, as illustrated in FIG. 8, the information output unit 16 displays the identification information of specialists a, b, and c on the screen. Also in this case, as illustrated in FIG. 8, the number of types of treatments of which attending doctor A has no experience may be displayed, or display may be performed according to the number of types of treatments of which attending doctor A has no experience (display of the degree of priority by the thickness of an arrow, display of the degree of priority by rearrangement of the displayed order, or the like).

By referring to a screen such as those in FIGs. 7 and 8, attending doctor A may find a specialist suitable for introduction to a patient (specialist capable of treating an injury or disease of the patient). Accordingly, attending doctor A may introduce a suitable doctor to the patient.

As described above in detail, according to the present first embodiment, the search condition acquisition unit 12 acquires an injury or disease name of a patient as a search condition (S10). The information extraction unit 14 refers to the medical examination history data (FIG. 4A) stored in the DB server 60, and stores, in a correspondence table (FIG. 4B), the information on the type of treatment in the record including the injury or disease name of the patient in association with the identification information of a doctor (S16). The information output unit 16 refers to the correspondence table, and outputs the identification information of a doctor for which information on the type of treatment satisfies a criterion (S20 to S28). Accordingly, the server 10 may output, to the doctor terminal 70, identification information of a doctor capable of treating an injury or disease of a patient. By checking the output information, a doctor (attending doctor A) may introduce a suitable doctor to the patient.

In local governments such as prefectures, cities, towns, and villages, a medical policy in which doctors provide services in cooperation with each other has been planned and implemented in order to enable residents to receive high-quality medical care at ease. In order to provide such services, doctors have to know the kind of treatment that other doctors are able to perform. In the present embodiment, since a doctor who intends to introduce another doctor to a patient may be provided with information on doctors capable of treating an injury or disease of the patient, cooperation between doctors may be smoothly performed.

In the present embodiment, data referred to by the information extraction unit 14 is the medical examination history data stored in the DB server 60. Accordingly, the types of treatments that each doctor is able to perform may be extracted from the data in which daily treatment actions are recorded. Therefore, time and effort may be saved as compared with a case where the types of treatments that each doctor is able to perform are recorded in a database.

In the present embodiment, the search condition acquisition unit 12 also acquires the identification information of a doctor (attending doctor A) who has input the search conditions as a search condition (S10). The information output unit 16 may set the "criterion" when determining the doctor of an output target to be that "the treatments of the selected doctor include a treatment other than the treatments of the doctor (attending doctor A) who has input the search conditions". By doing so, it is possible to output the identification information of a doctor capable of performing the treatment that the attending doctor A is not able to perform. Therefore, by checking the output information, the doctor (attending doctor A) may introduce, to a patient, a doctor capable of performing the treatment that attending doctor A himself/herself is not able to perform.

In the present embodiment, the information output unit 16 outputs the identification information of a doctor in a mode (thickness of an arrow or displayed order) according to the number of types of treatments that may be performed. Accordingly, a doctor to be preferentially introduced to a patient may be displayed in an easily understandable manner.

### (Modification Example 1)

Hereinafter, Modification Example 1 will be described.

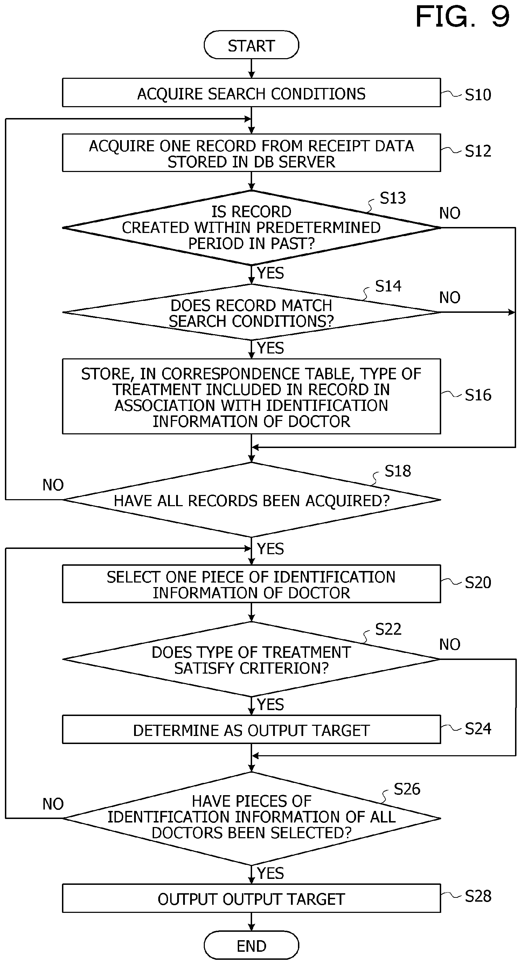

The present Modification Example 1 is different from the above embodiment in that the information extraction unit 14 creates a correspondence table using data obtained from medical examination history data created within a predetermined period in the past (for example, within the past year). In this case, for example, the server 10 executes the processing according to the flowchart in FIG. 9.

As may be seen by comparing FIGs. 9 and 5, the processing in FIG. 9 is different from the processing in FIG. 5 in that step S13 is executed between step S12 and step S14.

When the processing in FIG. 9 is started, first, in step S10, the search condition acquisition unit 12 acquires search conditions. Next, in step S12, the information extraction unit 14 acquires one record from the medical examination history data (FIG. 4A) stored in the DB server 60. Next, in step S13, the information extraction unit 14 determines whether the acquired record is a record created within a predetermined period in the past (for example, a record created within the past year). When the determination in step S13 is affirmative, the information extraction unit 14 proceeds to step S14. On the other hand, when the determination in step S13 is negative, the information extraction unit 14 proceeds to step S18.

The remaining processing is similar to the processing in FIG. 5.

As described above, in the present Modification Example 1, by performing the determination in step S13, a correspondence table may be created using only records created within a predetermined period in the past. Accordingly, information on the experience of a treatment that is currently not very often performed may be excluded when a correspondence table is created. Therefore, it is possible to output the identification information of a doctor more suitable for introduction to a patient.

The processing in step S13 may be performed after step S14. In this case, when the determination in step S13 is affirmative, the processing proceeds to step S16, and when the determination in step S13 is negative, the processing proceeds to step S18.

### (Modification Example 2)

Next, Modification Example 2 will be described.

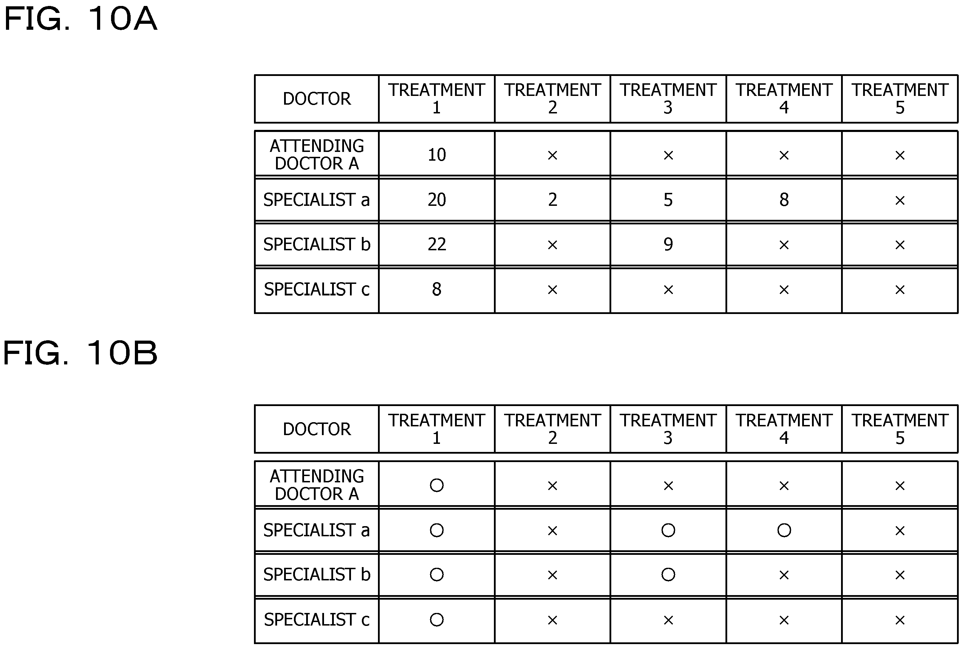

In the present Modification Example 2, when a correspondence table is created, as illustrated in FIG. 10A, the information extraction unit 14 counts the number of pieces of information on each type of treatment in the records (step S16 in FIG. 5). In a case where the correspondence table in FIG. 10A has been created, the information output unit 16 changes the fields in which the counted number is equal to or larger than a predetermined number (for example, three or more) to "o" and changes the other fields to "×" before the processing of step S20 and subsequent steps is started. Accordingly, a correspondence table such as that illustrated in FIG. 10B is created. The information output unit 16 executes processing similar to that in the above embodiment (S20 to S28) based on the correspondence table in FIG. 10B.

By doing so, in the present Modification Example 2, it is possible not to regard a type of treatment of which a doctor has little experience as a type of treatment that the doctor is able to perform. Accordingly, it is possible to output the identification information of a doctor more suitable as a candidate to be introduced to a patient.

### (Modification Example 3)

Next, Modification Example 3 will be described.

The present Modification Example 3 is a combination of the above Modification Example 1 and Modification Example 2. For example, when a correspondence table is created, the information extraction unit 14 uses records created within a predetermined period in the past (step S13 in FIG. 9). When a correspondence table is created, as illustrated in FIG. 10A, the information extraction unit 14 counts the number of pieces of information on each type of treatment in the records. The information output unit 16 changes the fields in the correspondence table in FIG. 10A in which the counted number is equal to or larger than a predetermined number (for example, three or more) to "o" and changes the other fields to "×" (FIG. 10B) before the processing of step S20 and subsequent steps is started. The information output unit 16 executes processing similar to that in the above embodiment (S20 to S28) based on the correspondence table in FIG. 10B.

By doing so, in the present Modification Example 3, a type of treatment of which a doctor has a lot of experience within a predetermined period in the past is regarded as a treatment that the doctor is able to perform. Accordingly, it is possible to output the identification information of a doctor more suitable as a candidate to be introduced to a patient.

### (Modification Example 4)

Next, Modification Example 4 will be described.

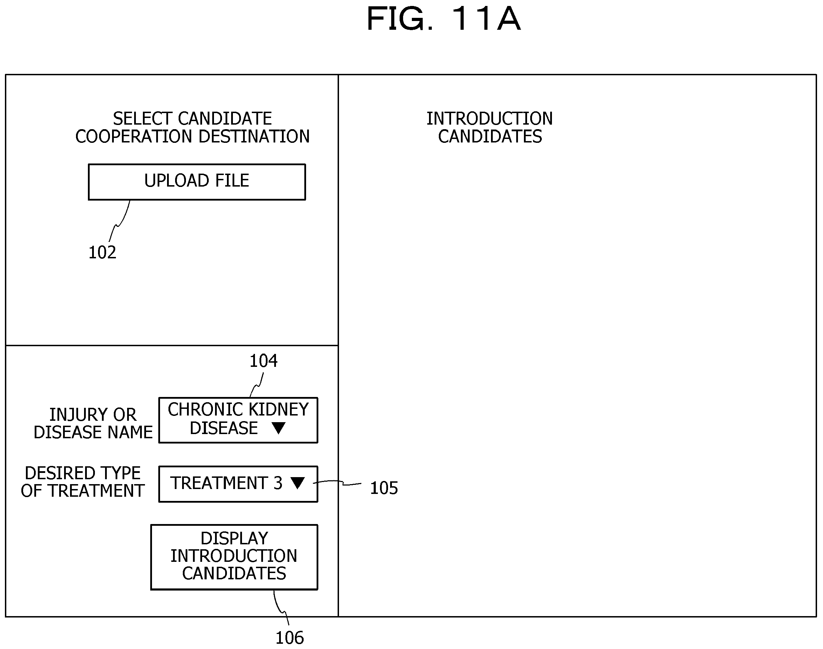

In the present Modification Example 4, for example, as illustrated in FIG. 11A, a drop-down list (pull-down) 105 with which a "desired type of treatment" is selectable is provided in the screen displayed on the display unit 193 of the doctor terminal 70. A doctor (attending doctor A) selects an injury or disease name of a patient from the drop-down list 104 and selects a type of treatment that a doctor desired to be introduced is able to perform from the drop-down list 105, and presses the button 106.

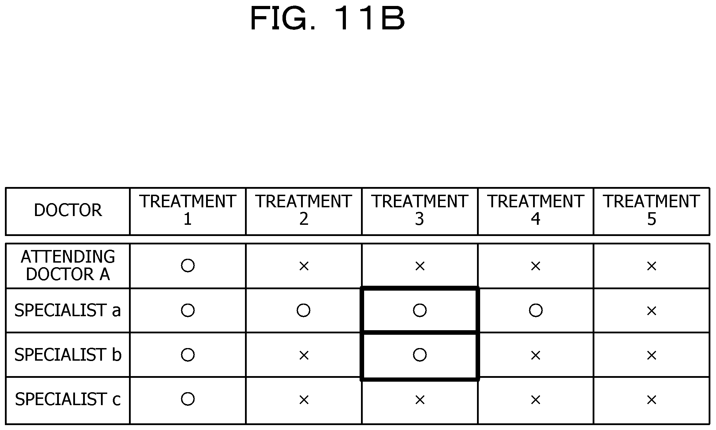

In this case, the information extraction unit 14 creates a correspondence table such as that illustrated in FIG. 11B by executing steps S12 to S18 in FIG. 5. As the "criterion" used in step S22, the information output unit 16 adopts that "the treatments of the selected doctor include the type of treatment desired by attending doctor A".

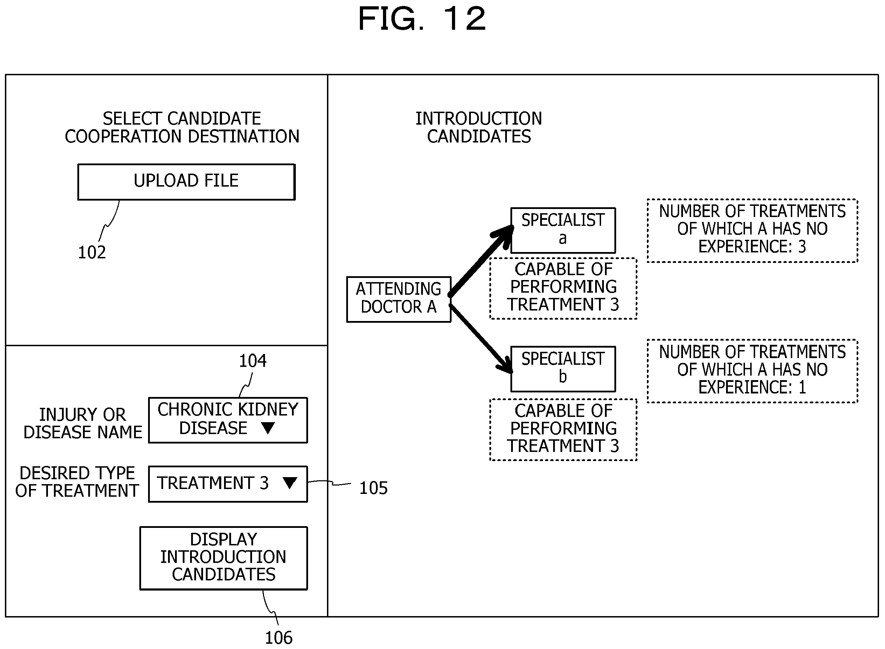

As in FIG. 11A, in a case where attending doctor A selects "treatment 3" as the desired type of treatment, the information output unit 16 determines, as output targets, specialists a and b capable of performing treatment 3 based on the correspondence table in FIG. 11B. Therefore, a screen such as that illustrated in FIG. 12 is displayed on the display unit 193 of the doctor terminal 70.

By doing so, in the present Modification Example 4, in a case where the condition of a doctor desired to be introduced to a patient is determined as that the doctor is capable of performing a predetermined treatment, it is possible to output the identification information of a doctor suitable as a candidate to be introduced to a patient.

### (Modification Example 5)

Next, Modification Example 5 will be described.

In the present Modification Example 5, similarly to the above Modification Example 4, a screen such as that in FIG. 11A is used. As the "criterion" used in step S22, "the treatments of the selected doctor include the type of treatment desired by attending doctor A" is adopted. In the present Modification Example 5, the way in which the information output unit 16 outputs identification information of a doctor is different from that in the above Modification Example 4.

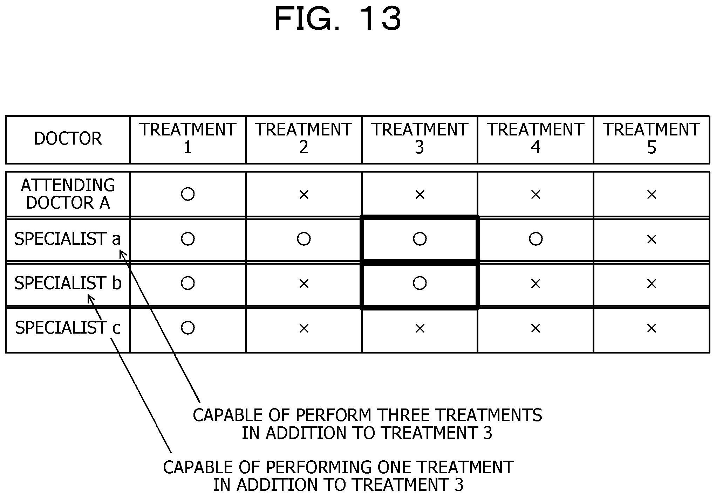

For example, as illustrated in FIG. 13, in a case where the specialists capable of performing the type of treatment desired by attending doctor A "treatment 3" are specialists a and b, the information output unit 16 preferentially outputs a doctor capable of performing less types of treatments among the types of treatments other than treatment 3.

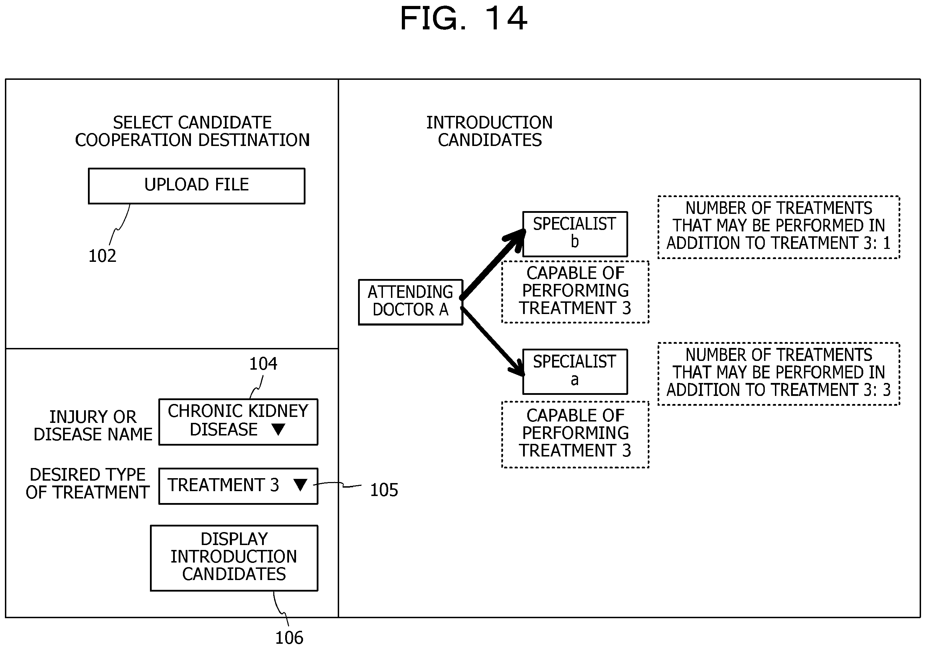

For example, in the case of FIG. 13, specialist a is capable of performing three types of treatments in addition to treatment 3, and specialist b is capable of performing one type of treatment in addition to treatment 3. Therefore, in this case, the information output unit 16 preferentially outputs specialist b. For example, as illustrated in FIG. 14, the information output unit 16 changes the display mode such as the thickness of an arrow or the displayed order so that the possibility of selecting specialist b is higher than the possibility of selecting specialist a.

By doing so, in the present Modification Example 5, it is possible to suppress concentration of patients on a doctor capable of performing many types of treatments.

### (Modification Example 6)

Next, Modification Example 6 will be described.

In the present Modification Example 6, when the identification information of a doctor is output in the above embodiment and Modification Examples 1 to 5, the output is performed according to a distance between the address of a patient and the location of the doctor (place of treatment).

For example, in a case where a screen such as that illustrated in FIG. 8 is output, the information output unit 16 calculates a distance between the address of a patient and the location of a doctor to be output (place of treatment). The information output unit 16 may acquire information on the address of a patient from an electronic medical record or the like, or may provide an input field for the address of a patient on the screen in FIG. 8 and acquire the information input to the input field. The information output unit 16 may search for and acquire the location of a doctor (place of treatment) on the Web, or may acquire the location from a doctor DB or the like managed in advance in the DB server 60.

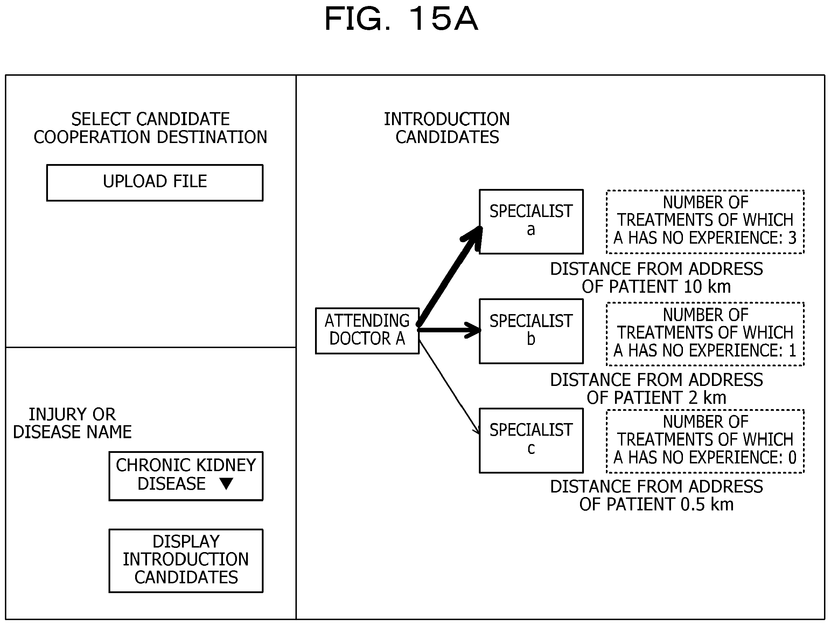

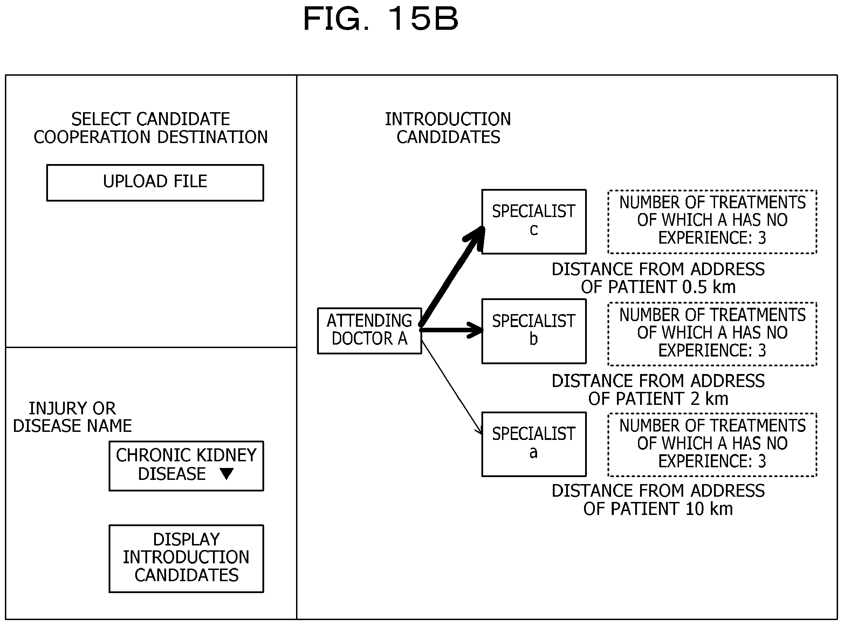

The information output unit 16 performs output according to the calculated distance. For example, as illustrated in FIG. 15A, the calculated distance may be displayed near the identification information of a doctor. As illustrated in FIG. 15B, in a case where the numbers of types of treatments that specialists a, b, and c are able to perform are the same, the identification information of the doctors may be displayed by being arranged in ascending order of distance. A displayed order or a display mode of the identification information of doctors may be determined based on an index value obtained by substituting a distance and the number of types of treatments that may be performed into a predetermined calculation expression (weighting function or the like).

The location of attending doctor A (place of treatment) may be used instead of the address of a patient. This is because there is a high possibility that the address of a patient and the location of attending doctor A are close to each other.

### (Other Modification Examples)

In the above first embodiment and Modification Examples 1 to 6, a case has been described in which attending doctor A inputs (uploads) candidate cooperation destinations on the screen in FIG. 6 or the like, but this is not the only case. For example, candidate cooperation destinations do not have to be input. As conditions for narrowing down candidate cooperation destinations, a region, the age and sex of a doctor, and the like may be input.

In the above first embodiment and Modification Examples 1 to 6, a case has been described in which data referred to by the information extraction unit 14 is medical examination history data, but this is not the only case. For example, other data may be used as long as information on an injury or disease treated by a doctor and the type of performed treatment are stored in the data. The information extraction unit 14 may refer to a database in which information on injuries or diseases that may be treated by doctors, and types of treatments that may be performed by doctors are acquired from a result of a questionnaire for doctors, a website of a hospital, or the like, and summarized.

### <<Second Embodiment>>

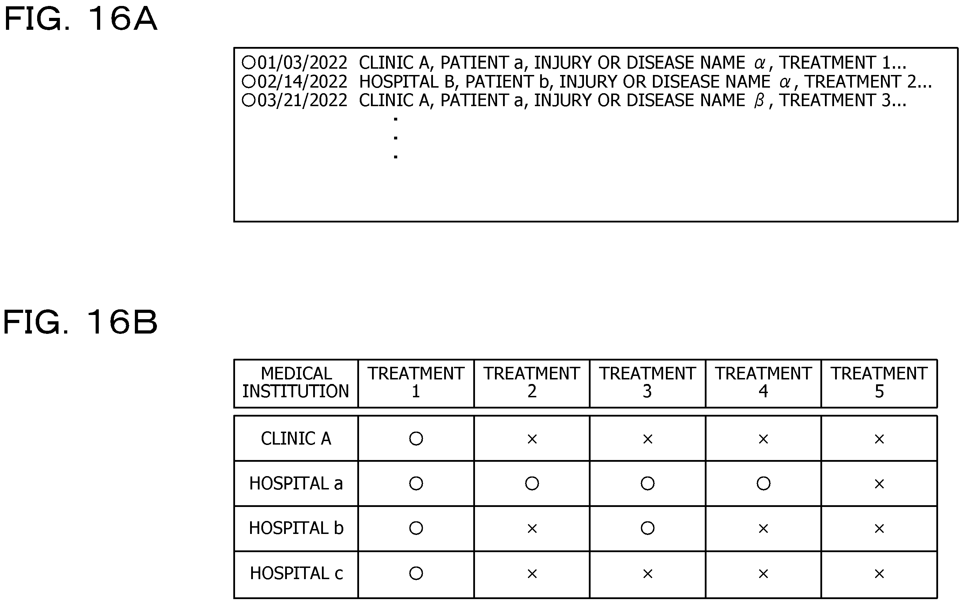

Next, a second embodiment will be described based on FIGs. 16A to 17B. In the present second embodiment, the DB server 60 in FIG. 1 includes medical examination history data such as that illustrated in FIG. 16A instead of the medical examination history data in FIG. 4A. The medical examination history data in FIG. 16A is data included in a national health insurance (national insurance) database or a diagnosis procedure combination (DPC) database. The national insurance database is a database for managing and utilizing medical information or the like by receiving a commission of an insurer. The DPC database is a medical fee database, and includes medical institution information, patient information, medical information, surgery information, information on an examination, treatment, or drug performed or used for a patient, and the like.

Each record (row) of the medical examination history data in FIG. 16A includes information such as year, month, and day of medical examination (treatment), identification information of a medical institution that has performed the medical examination (treatment), identification information of a patient, an injury or disease name, and a type of the performed treatment. For example, in the medical examination history data, in association with the identification information of a medical institution, injury or disease treatment information including information on the type of injury or disease for which the medical institution has an experience of treatment and information on the type of treatment, and information on the date on which the treatment was performed are stored.

Hereinafter, description will be given for the function of each unit of the server 10 (the search condition acquisition unit 12, the information extraction unit 14, and the information output unit 16 in FIG. 3) in the present second embodiment.

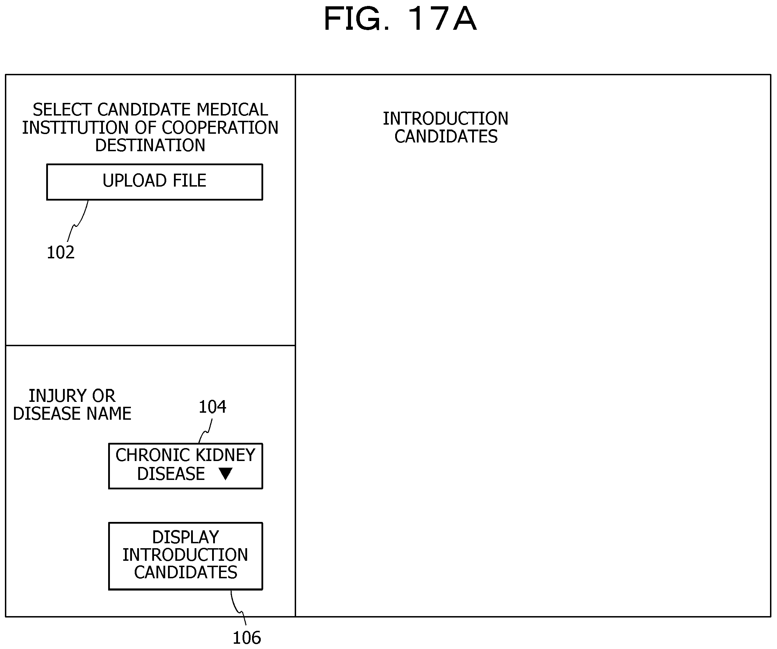

The search condition acquisition unit 12 acquires the search conditions input in the doctor terminal 70 and notifies the information extraction unit 14 of the search conditions. The search conditions include information on a medical institution to which a doctor who has input the search condition belongs, information on a medical institution of a cooperation destination, and an injury or disease name of a patient. For example, attending doctor A uploads a file in which the identification information of medical institutions of cooperation destinations is stored by pressing the "upload file" button 102 on the screen in FIG. 17A. Attending doctor A selects an injury or disease name of a patient from the drop-down list (pull-down) 104, and presses the "display introduction candidates" button 106. In this case, the doctor terminal 70 transmits, to the search condition acquisition unit 12 of the server 10, a list of the identification information of medical institutions of cooperation destinations included in the uploaded file and the information on the selected injury or disease name (first injury or disease information). The doctor terminal 70 also transmits the identification information of a medical institution to which attending doctor A belongs (clinic A) to the search condition acquisition unit 12 of the server 10. The search condition acquisition unit 12 acquires, as search conditions, the list of the identification information of medical institutions of cooperation destinations and the identification information of a medical institution to which attending doctor A belongs (clinic A), and the information on the selected injury or disease name, which are transmitted from the doctor terminal 70.

When information on a medical institution and an injury or disease name are input as search conditions, the information extraction unit 14 searches for and extracts data (record) created when the medical institution of a search target performed the treatment for the injury or disease of a search target, from the medical examination history data in FIG. 16A. The information extraction unit 14 creates a correspondence table (see FIG. 16B) based on the contents of the extracted record.

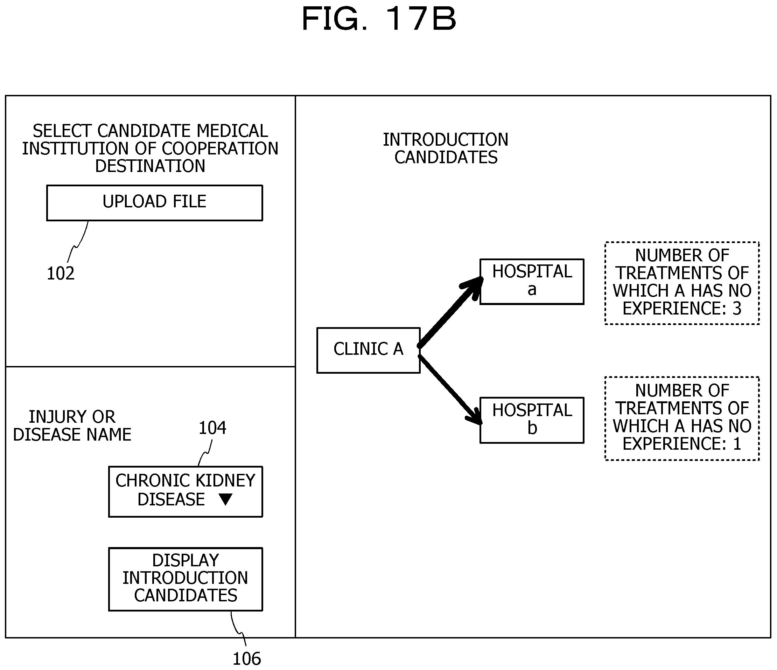

Based on the correspondence table created by the information extraction unit 14, the information output unit 16 determines whether the type of treatment that has been performed in each medical institution of a cooperation destination (type of experienced treatment) satisfies a criterion. The information output unit 16 generates a screen for displaying information on a medical institution of a cooperation destination satisfying the criterion, and transmits (outputs) the screen to the doctor terminal 70. For example, in a case where the "criterion" is that "the treatments that have been performed in a medical institution include a treatment other than the treatments that have been performed in the medical institution to which the doctor (attending doctor A) who has input the search conditions belongs (clinic A)", hospitals a and b are output targets according to FIG. 16B. In this case, as illustrated in FIG. 17B, the information output unit 16 displays the identification information of hospitals a and b as introduction candidates on the screen. The information output unit 16 may simply display only the identification information of hospitals a and b, but as illustrated in FIG. 17B, the information output unit 16 may display the number of types of treatments of which the medical institution to which attending doctor A belongs (clinic A) has not experience, or may perform display according to the number of types of treatments of which clinic A has no experience. As the display according to the number of types of treatments of which clinic A has no experience, for example, as illustrated in FIG. 17B, the degree of priority (degree to which priority is to be given as an introduction candidate) may be represented by the thickness of an arrow. The degree of priority may be represented by rearranging the displayed order of identification information of medical institutions in descending order of the number of types of treatments of which clinic A has no experience.

In a case where the "criterion" is that "the medical institution is capable of performing at least one treatment", a screen similar to that in FIG. 8 may be displayed.

By referring to a screen such as that in FIG. 17B, attending doctor A may find a medical institution suitable for introduction to a patient (medical institution capable of treating an injury or disease of the patient). Accordingly, attending doctor A may introduce a suitable medical institution to the patient.

As described above in detail, according to the present second embodiment, the search condition acquisition unit 12 acquires an injury or disease name of a patient as a search condition. The information extraction unit 14 refers to the medical examination history data (FIG. 16A) stored in the DB server 60, and stores, in a correspondence table (FIG. 16B), the information on the type of treatment in the record including the injury or disease name of the patient in association with the identification information of a medical institution. The information output unit 16 refers to the correspondence table, and outputs the identification information of a medical institution for which information on the type of treatment satisfies a criterion (FIG. 17B). Accordingly, the server 10 may output, to the doctor terminal 70, identification information of a medical institution capable of treating an injury or disease of a patient. By checking the output information, a doctor (attending doctor A) may introduce a suitable medical institution to the patient.

In the above second embodiment, Modification Examples 1 to 6 and other modification examples described in the above first embodiment may be employed as appropriate.

### <<Third Embodiment>>

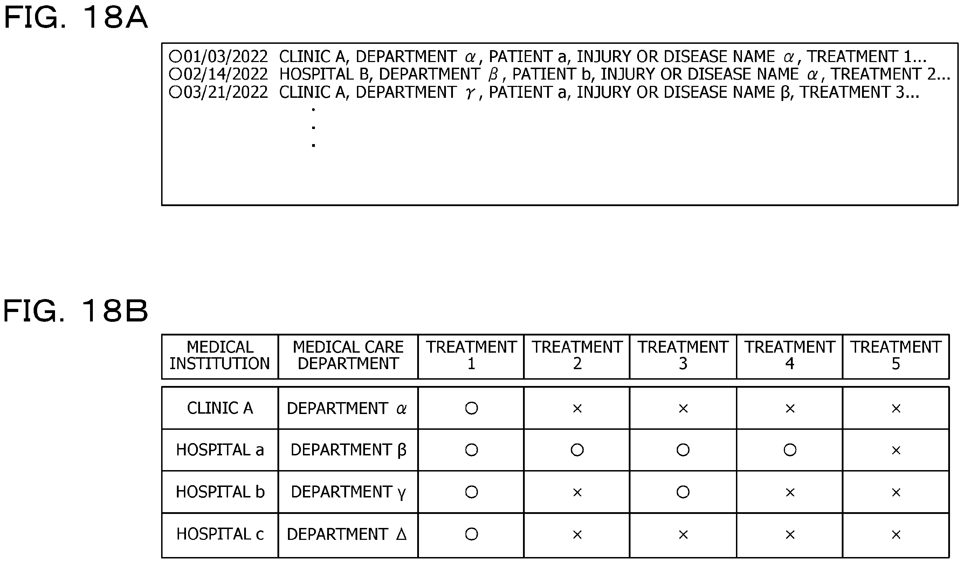

Next, a third embodiment will be described based on FIGs. 18A and 18B. In the present third embodiment, the DB server 60 in FIG. 1 includes medical examination history data such as that illustrated in FIG. 18A instead of the medical examination history data in FIG. 4A. Each record (row) of the medical examination history data in FIG. 18A includes information such as year, month, and day of medical examination (treatment), identification information of a medical institution and a medical care department that have performed the medical examination (treatment), identification information of a patient, an injury or disease name, and a type of the performed treatment. For example, in the medical examination history data, in association with the identification information of a medical care department of a medical institution, injury or disease treatment information including information on the type of injury or disease for which the medical care department has an experience of treatment and information on the type of treatment, and information on the date on which the treatment was performed are stored.

The function of each unit of the server 10 (the search condition acquisition unit 12, the information extraction unit 14, and the information output unit 16 in FIG. 3) of the present third embodiment is similar to that in the above second embodiment. However, the present third embodiment is different in that the processing for each medical institution performed in the above second embodiment is processing for each combination of a medical institution and a medical care department.

For example, when information on a combination of a medical institution and a medical care department and an injury or disease name are received as search conditions, the information extraction unit 14 searches for and extracts data (record) created when the medical care department of the medical institution of a search target performed the treatment for the injury or disease of a search target, from the medical examination history data in FIG. 18A. As a result, the information extraction unit 14 creates a correspondence table such as that illustrated in FIG. 18B.

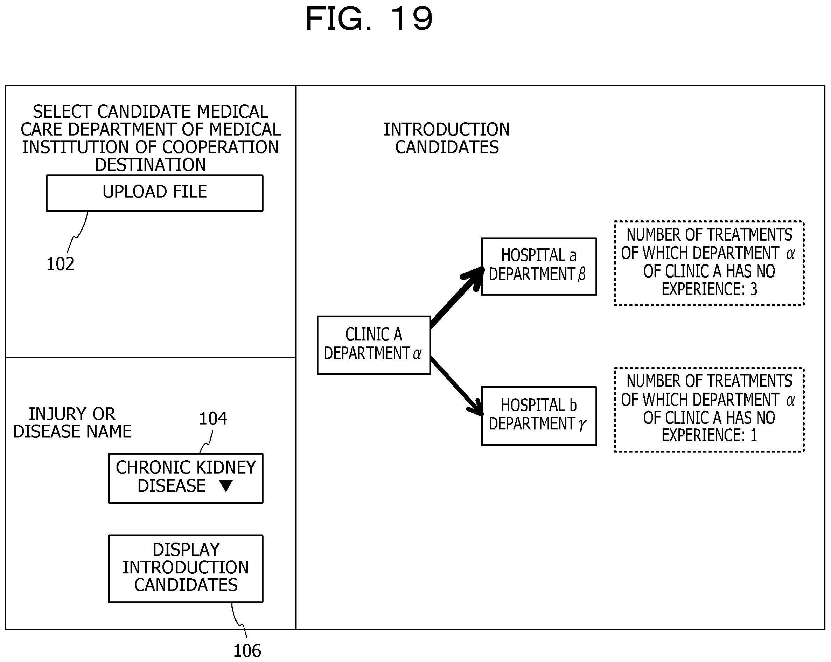

Based on the correspondence table in FIG. 18B, the information output unit 16 determines whether the type of treatment that has been performed in the medical care department of each medical institution of a cooperation destination satisfies a criterion, and generates a screen for displaying the determination result (see FIG. 19).

By referring to a screen such as that in FIG. 19, attending doctor A may find a medical care department of a medical institution suitable for introduction to a patient (medical care department of a medical institution capable of treating an injury or disease of the patient). Accordingly, attending doctor A may introduce a suitable medical care department to the patient.

As described above in detail, according to the present third embodiment, the search condition acquisition unit 12 acquires an injury or disease name of a patient as a search condition. The information extraction unit 14 refers to the medical examination history data (FIG. 18A) stored in the DB server 60, and stores, in a correspondence table (FIG. 18B), the information on the type of treatment in the record including the injury or disease name of the patient in association with the identification information of a medical care department of a medical institution. The information output unit 16 refers to the correspondence table, and outputs the identification information of a medical care department of a medical institution for which information on the type of treatment satisfies a criterion (FIG. 19). Accordingly, the server 10 may output, to the doctor terminal 70, identification information of a medical care department of a medical institution capable of treating an injury or disease of a patient. By checking the output information, a doctor (attending doctor A) may introduce a suitable medical care department of a medical institution to the patient.

In the above third embodiment, Modification Examples 1 to 6 and other modification examples described in the above first embodiment may be employed as appropriate.

The above processing functions may be realized by a computer. In this case, a program is provided that describes the processing contents of the functions to be included in a processing apparatus. By causing the computer to execute the program, the above processing functions are realized in the computer. The program that describes the processing contents may be recorded in a computer-readable storage medium (except for a carrier wave).

In a case where a program is distributed, for example, the program is sold in the form of a portable-type storage medium such as a Digital Versatile Disc (DVD), a compact disc read-only memory (CD-ROM), or the like in which the program is recorded. The program may be stored in a storage device of a server computer and transferred from the server computer to another computer via a network.

For example, the computer executing a program stores, in its own storage device, the program recorded in the portable-type storage medium or the program transferred from the server computer. The computer reads the program from its own storage device and executes processing in accordance with the program. The computer may read the program directly from the portable-type storage medium and execute processing in accordance with the program. Each time when a program is transferred from the server computer, the computer may execute processing in accordance with the received program.

The above-described embodiments are examples of a preferred embodiment of the present disclosure. However, these are not the only examples, and various modifications may be made without departing from the gist of the present disclosure.

The following appendices are further disclosed in relation to the description of the above embodiments and modification examples.

## Claims

1. A medical information providing method implemented by a computer, the medical information providing method comprising:
acquiring first injury-or-disease information that indicates a type of an injury-or-disease;
accessing a storage device that stores, for each provider of a plurality of providers each of which provides medical service, identification information of the provider, and injury-or-disease treatment information, the injury-or-disease treatment information including information on a type of an injury-or-disease for which the provider has an experience of treatment or that the provider is able to treat, and information on a type of treatment;
extracting, for each provider of the plurality of providers, from the storage device, the injury-or-disease treatment information that includes information on a type of injury-or-disease corresponding to the first injury-or-disease information; and
in a case where the information on a type of treatment included in the injury-or-disease treatment information extracted for each provider of the plurality of providers satisfies a given criterion, outputting the identification information of the provider.

2. The medical information providing method according to claim 1,
wherein information is recorded in the storage device each time the provider provides medical service to a patient.

3. The medical information providing method according to claim 1, wherein
the acquiring of the first injury-or-disease information includes acquiring identification information of a first provider, and
the outputting of the identification information of the provider is performed in a case where the information on a type of treatment included in the injury-or-disease treatment information extracted for each provider of the plurality of providers includes information other than information on a type of treatment associated with both of identification information of the first provider and the first injury-or-disease information in the storage device.

4. The medical information providing method according to claim 1, wherein
the outputting outputs identification information of the provider in a mode according to a number of the type of treatment included in the injury-or-disease treatment information extracted for each piece of identification information of the provider.

5. The medical information providing method according to claim 1, wherein
the storage device stores, for each provider of the plurality of providers, in association with the identification information of the provider, injury-or-disease treatment information that includes information on a type of an injury-or-disease for which the provider has an experience of treatment, information on a type of treatment, and a date on which a treatment was performed,
the extracting extracts, for each provider of the plurality of providers, from the injury-or-disease treatment information that includes information on a type of injury-or-disease corresponding to the first injury-or-disease information, the injury-or-disease treatment information in which the date on which a treatment was performed is included within a predetermined period in a past.

6. The medical information providing method according to claim 1,
wherein the outputting of the identification information of the provider is performed in a case where a predetermined number or more of pieces of the information on a type of treatment included in the injury-or-disease treatment information extracted for each provider satisfy a criterion.

7. The medical information providing method according to claim 1, wherein
the storage device stores, for each provider of the plurality of providers, in association with the identification information of the provider, injury-or-disease treatment information that includes information on a type of an injury-or-disease for which the provider has an experience of treatment, information on a type of treatment, and a date on which a treatment was performed,
the extracting extracts, for each provider of the plurality of providers, from the injury-or-disease treatment information that includes information on a type of injury-or-disease corresponding to the first injury-or-disease information, the injury-or-disease treatment information in which the date on which a treatment was performed is included within a predetermined period, and
the outputting of the identification information of the provider is performed in a case where a predetermined number or more of pieces of the information on a type of treatment included in the injury-or-disease treatment information extracted for each provider satisfy a criterion.

8. The medical information providing method according to claim 1, wherein
the acquiring includes acquiring information on a first type of treatment, and
the outputting of the identification information of the provider is performed in a case where the information on a type of treatment included in the injury-or-disease treatment information extracted for each provider of the plurality of providers includes the information on a first type of treatment.

9. The medical information providing method according to claim 8,
wherein the outputting preferentially outputs identification information of a provider with a smaller number of types of treatments included in the extracted injury-or-disease treatment information.

10. The medical information providing method according to any one of claims 1 to 9,
wherein the outputting of the identification information of the provider includes outputting information on a distance between a place where the provider performs a treatment and a first position related to a patient, and/or
outputting the identification information of the provider in a mode according to the information on the distance.

11. A medical information providing program which, when executed by a computer, cause the computer to perform processing, the processing comprising:
acquiring first injury-or-disease information that indicates a type of an injury-or-disease;
accessing a storage device that stores, for each provider of a plurality of providers each of which provides medical service, identification information of the provider, and injury-or-disease treatment information, the injury-or-disease treatment information including information on a type of an injury-or-disease for which the provider has an experience of treatment or that the provider is able to treat, and information on a type of treatment;
extracting, for each provider of the plurality of providers, from the storage device, the injury-or-disease treatment information that includes information on a type of injury-or-disease corresponding to the first injury-or-disease information; and
in a case where the information on a type of treatment included in the injury-or-disease treatment information extracted for each provider of the plurality of providers satisfies a given criterion, outputting the identification information of the provider.

12. An information processing apparatus comprising:
an acquisition unit configured to acquire first injury-or-disease information that indicates a type of an injury-or-disease;
an extraction unit configured to
access a storage device that stores, for each provider of a plurality of providers each of which provides medical service, identification information of the provider, and injury-or-disease treatment information, the injury-or-disease treatment information including information on a type of an injury-or-disease for which the provider has an experience of treatment or that the provider is able to treat, and information on a type of treatment, and
extract, for each provider of the plurality of providers, from the storage device, the injury-or-disease treatment information that includes information on a type of injury-or-disease corresponding to the first injury-or-disease information; and
an output unit configured to output, in a case where the information on a type of treatment included in the injury-or-disease treatment information extracted for each provider of the plurality of providers satisfies a given criterion, the identification information of the provider.
